**Europäisches Patentamt**

**European Patent Office**

**Office européen des brevets**

(19)

(11) Publication number : **0 465 047 A2**

(12) # EUROPEAN PATENT APPLICATION

(21) Application number : **91305545.5**

(22) Date of filing : **19.06.91**

(51) Int. Cl.⁵ : **C12N 15/71, C12N 15/67**

(30) Priority : **25.06.90 US 542541**

(43) Date of publication of application :
**08.01.92 Bulletin 92/02**

(84) Designated Contracting States :
**AT BE CH DE DK ES FR GB GR IT LI NL SE**

(71) Applicant : **ELI LILLY AND COMPANY**
**Lilly Corporate Center**
**Indianapolis Indiana 46285 (US)**

(72) Inventor : **Belagaje, Rama M.**
**7821 Mohawk Lane**
**Indianapolis, Indiana 46260 (US)**

(74) Representative : **Hudson, Christopher Mark et al**
**Erl Wood Manor**
**Windlesham Surrey GU20 6PH (GB)**

(54) **Modified tryptophan promoters.**

(57)    The present invention provides a tryptophan operon transcriptional activating sequence comprising a -10 consensus sequence and a wild type repressor binding site. The transcriptional activating sequences, derived from the tryptophan promoter-operator region, are regulatable and enable improved expression levels of an operably linked structural gene.

EP 0 465 047 A2

This invention is in the field of molecular biology. The invention provides novel transcriptional activating sequences that provide an increased level of transcription of an operably linked gene.

The level of gene expression in a microorganism is primarily dependent on the efficiency of transcription. Factors that contribute to the efficiency of transcription include the promoter strength, the nucleotide sequence in the vicinity of the ribosome binding site and the translational start site, the presence of a transcription terminator, the plasmid copy number, the choice of bacterial host and the way in which the transformed bacterial host is grown.

Transcriptional promoters are segments of DNA that direct RNA polymerase binding and initiation of transcription. Various promoter sites recognized by Escherichia coli RNA polymerase have been structurally characterized (Rosenberg et al., 1979, Ann. Rev. Genet. 13:319; Stebenlist et al., 1980, Cell 20:269). Comparison of these sequences has revealed two regions of homology, the -10 region, with a consensus sequence TATAAT, and the -35 region, with a consensus sequence TTGACA (Pribnow et al., 1975, Proc. Natl. Acad. Sci. U.S.A. 79:1069; Gilbert et al., 1976, in RNA Polymerase, Losick and Chamberlin eds., Cold Spring Harbor Laboratories, Cold Spring Harbor, New York).

Most nucleotide changes that adversely affect the transcriptional promoter activity decrease the extent of homology with the consensus sequence. The few nucleotide changes that increase the promoter activity increase the extent of homology with the consensus -10 and -35 regions. For example, the Escherichia coli lipoprotein promoter, that contains a consensus -35 region, was altered to provide a consensus sequence in the -10 region. This change provided improved promoter efficiency as measured by β-galactosidase activity (Inouye et al., 1985, Nucleic Acids Res. 13:3101). Similarly a hybrid promoter, derived from the E.coli tryptophan operon and the E.coli lac promoter, with the consensus -10 and -35 sequences has been shown to be stronger than either one of the parental promoters (DeBoer et al., 1983, Proc. Natl. Acad. Sci. U.S.A. 80:21; Amann et al., 1983, Gene 25:167).

It is important to be able to regulate the transcription of a gene since constitutive expression may often be detrimental to the host cell. In Escherichia coli various promoters including the E. coli lac UV5 promoter, the pL promoter-operator transcriptional activating sequence from the E. coli bacteriophage lambda and the E. coli tryptophan promoter have been used as regulators of gene transcription (Goeddel et al., 1979, Nature 281:544; Edman et al., 1981, Nature 291:503; Roberts et al., 1979, Proc. Natl. Acad. Sci. U.S.A 76:5596; Edge et al., 1981, Nature 292:756; Remaut et al., 1981, Gene 15:81 and Remaut et al., 1983, Gene 22:103). These regulatable transcription promoters are useful in controlling the transcription of an operably linked gene.

The Escherichia coli tryptophan operon promoteroperator sequence contains a regulatable promoter from which initiation is known to be repressed to very low levels in the presence of tryptophan. Trytophan and the tryptophan repressor protein form a complex which binds to the repressor binding site within the tryptophan promoter-operator region. The bound complex excludes RNA polymerase from binding to the promoter sequence preventing transcription. In the absence of tryptophan, RNA polymerase can bind to the promoter sequence, and transcription can occur. Thus, the tryptophan promoter has the advantage of being controllable in E. coli, a characteristic that is highly desirable where large scale microbial fermentation is concerned.

In the production of polypeptides by recombinant DNA methods, a primary objective is to produce large quantities of the polypeptide of interest in an efficient manner. The present invention meets this objective by providing transcriptional activating sequences that enable regulatable production of a polypeptide of interest in quantities greater than those attainable using the wild type tryptophan transcriptional activating sequences.

The present invention provides novel DNA compounds useful in recombinant DNA technology. These DNA compounds, called transcriptional activating sequences, promote transcription of DNA. The novel transcriptional activating sequences are derived from the Escherichia coli tryptophan operon promoter-operator region (trp promoter) and enable efficient and regulatable expression of an operably linked gene.

The present invention includes recombinant DNA expression vectors comprising the novel transcriptional activating sequence, a translational activating sequence and an operably linked DNA sequence that codes for a functional polypeptide. When introduced into the appropriate host cell and cultured under the appropriate conditions these DNA expression vectors generate a translatable mRNA transcript.

The novel transcriptional activating sequence was constructed by modifying the trp promoter to include a -10 consensus sequence. As noted above, the -10 consensus sequence has been shown to provide improved promoter efficiency. However, the mere introduction of a -10 consensus sequence in the trp promoter would result in nucleotide changes within the tryptophan repressor binding region of the trp promoter. Various studies involving operator-repressor interactions suggest that mutations in the operator region affect the affinity for repressor binding (Maniatis, et al., 1978, Cell 5:109; Ptasne et al., 1976, Science 194:156; Bennett et al., 1978, J. Mol. Biol. 121:179; Oxender et al., Current Status of Tryptophan Repressor Structure-Function Studies in Protein Structure, Folding and Design (P. Oxender ed. 1987). The present invention avoids this problem by providing an efficient and regulatable tryptophan operon transcriptional activating sequence which comprises

EP 0 465 047 A2

a -10 consensus sequence and a wild type repressor binding site.

For the purpose of the present invention, as disclosed and claimed herein, the following terms are as defined below:

ApR - the ampicillin resistant phenotype or gene conferring the same.

Functional Polypeptide - A recoverable, biologically active heterologous or homologous polypeptide or precursor, a recoverable bioactive polypeptide comprised of a heterologous polypeptide and a partial or whole homologous polypeptide, or a recoverable bioinactive polypeptide containing a heterologous polypeptide and a bio-inactivating polypeptide which can be specifically cleaved or a bioinactive polypeptide that can be converted to a bioactive peptide by refolding or other chemical treatments known to those skilled in the art.

Promoter - a DNA sequence that directs transcription of DNA into RNA.

Recombinant DNA Expression Vector - any recombinant DNA cloning vector into which a promoter has been incorporated.

Recombinant DNA Cloning Vector - any agent including, but not limited to, recombinant plasmids, bacteriophages, and viruses, consisting of a DNA molecule to which one or more additional DNA segments can or have been added.

Structural Gene - any DNA sequence that encodes a functional polypeptide, inclusive of that DNA encoding the start and stop codons.

TAS - Transcriptional activating sequence.

Transcriptional Activating Sequence - a DNA sequence, such as a promoter, that directs the transcription of DNA in a regulatable fashion.

Translational Activating Sequence - a DNA sequence, including the ribosome binding site and translational start codon, such as 5′-ATG-3′, but not including any sequences downstream from the start codon, that provides for the initiation of translation of a mRNA transcript into a peptide or polypeptide.

Figure 1 presents the DNA sequences of the wild type and modified tryptophan transcriptional activating sequences.

Figure 2 is a restriction and function map of pK01S.

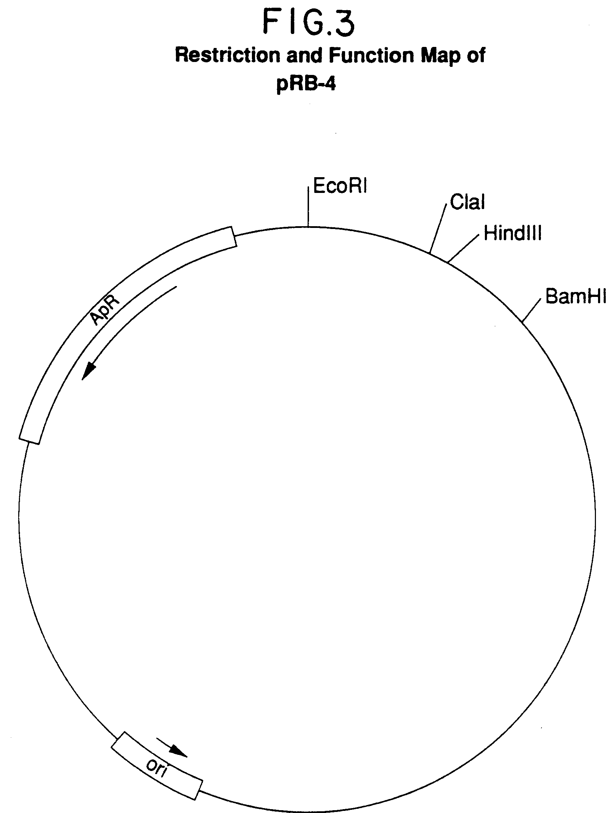

Figure 3 is a restriction and function map of pRB-4.

Figure 4 is a restriction and function map of pRB4-23.

The present invention provides a novel tryptophan operon transcriptional activating sequence comprising a -10 consensus sequence and an intact repressor binding site. The transcriptional activating sequence, derived from the Escherichia coli tryptophan operon, is regulatable and provides increased transcription of an operably linked gene.

A preferred embodiment of the present invention includes a recombinant DNA expression vector which sequentially comprises

a) a tryptophan operon transcriptional activating sequence comprising a -10 consensus sequence and a wild type repressor binding site,

b) a translational activating sequence, and

c) a DNA sequence that codes for a functional polypeptide; such that sequences a and b are positioned for the expression of sequence c.

Another preferred embodiment of the invention includes a tryptophan operon transcriptional activating sequence of the structure:

$$5\,{}'\text{-GTTGACAACGAACTAGTTAACTAGTATAATAGTACGCA-}3\,{}'$$
$$| | | | | | | | | | | | | | | | | | | | | | | | | | | | | | | | | | | | | |$$
$$3\,{}'\text{-CAACTGTTGCTTGATCAATTGATCATATTATCATGCGT-}5\,{}',$$

wherein

A is deoxyadenyl,

C is deoxycytidyl,

G is deoxyguanyl, and

T is thymidyl.

A preferred embodiment of the present invention also provides a recombinant DNA expression vector that sequentially comprises

a) a tryptophan operon transcriptional activating sequence of the structure:

3

```
5'-GTTGACAACGAACTAGTTAACTAGTATAATAGTACGCA-3'
   ||||||||||||||||||||||||||||||||||||||||
3'-CAACTGTTGCTTGATCAATTGATCATATTATCATGCGT-5'
```

b) a translational activating sequence and
c) a DNA sequence that codes for a functional polypeptide; such that sequences a and b are positioned for the expression of sequence c.

Host cells containing the novel recombinant DNA vectors are also provided.

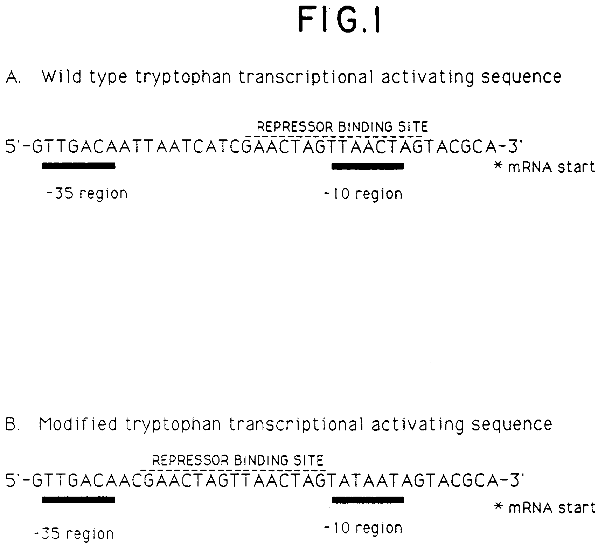

The novel transcriptional activating sequence of the present invention is derived from the Escherichia coli tryptophan promoter. The wild type tryptophan promoter, shown in Figure 1A, contains a consensus -35 region and a nonconsensus -10 region. The operon also contains a repressor binding site. When the repressor/tryptophan complex binds the repressor binding sites the transcription from the tryptophan promoter is repressed.

The wild type tryptophan promoter has a -35 consensus sequence but no -10 consensus sequence. Also, the 17 base pair sequence of the repressor binding site of the tryptophan promoter extends over the -10 region. Therefore, nucleotide substitutions introduced into the -10 region to provide a consensus sequence might affect the repressor binding such that regulation of the transcription from the promoter would be diminished or lost. However, the present invention completely avoids these deleterious nucleotide substitutions by moving the 17 nucleotide repressor towards the -35 region so that the repressor binding site occupies the region between the -10 and -35 domains (Figure 1B). Movement of the repressor binding site sequence allows the introduction of a consensus sequence in the -10 region without introducing a nucleotide substitution in the binding site. Surprisingly, the shifting of the repressor binding site does not affect the ability of this transcriptional activating sequence to be regulated in the presence of tryptophan. Also, the introduction of the consensus sequence in the -10 region provides an increased level of transcription.

The novel transcriptional activating sequence can be constructed from single stranded deoxyoligonucleotides by procedures well known in the art. The single stranded deoxyoligonucleotides can be synthesized with commercially available instruments such as the 380B DNA synthesizer marketed by Applied Biosystems (850 Lincoln Center Drive, Foster City CA 94404), which utilizes phosphoramidite chemistry. Other procedures for synthesizing DNA are also known in the art. The modified phosphotriester method of synthesizing single strand DNA is described in Itakura et al., 1977, Science 198:1056 and Crea et al., 1978, Proc. Nat. Acad. Sci. USA 75:5765. In addition, a preferred method of synthesizing DNA is disclosed in Hsiung et al., 1983, Nucleic Acid Research 11:3227 and Narang et al., 1980, Methods in Enzymology 68:90.

The plasmid pRB4-23 is illustrative of the recombinant DNA vectors of the present invention. Plasmid pRB4-23 was designed to place the expression of the Escherichia coli galactokinase gene (GalK) under the control of the novel transcriptional activating sequence of the present invention.

Plasmid pRB4-23 was constructed as follows. The 4.34 kb EcoRI-ClaI restriction fragment was isolated from the plasmid pBR322 (available from Bethesda Research Laboratories, P.O. Box 6009, Gaithersburg, MD 20877) and simultaneously ligated with the 229 base pair EcoRI-HincII restriction fragment of pNM608 and the 58 base pair synthetic DNA fragment shown in Example IC. The resulting plasmid was called pRB4-4. Alternatively, the 229 base pair EcoRI-HincII restriction fragment can be isolated from the plasmid pCZ20. Plasmid pCZ20 is disclosed and claimed in U.S. Patent 4,738,921, the entire teaching of which is incorporated herein by reference. This restriction fragment comprises DNA corresponding to the region located upstream of the -35 region of the tryptophan promoter. The 58 base pair synthetic DNA fragment includes the modified tryptophan operon transcriptional activating sequence, a transcription initiation site and an untranslated leader sequence, provided on a HincII/ClaI restriction fragment. The structure of this 58 base pair synthetic sequence is:

```
                                                    *
5'-GACAACGAACTAGTTAACTAGTATAATAGTACGCAAGTTCACGTAAAAA
   |||||||||||||||||||||||||||||||||||||||||||||||||
3'-CTGTTGCTTGATCAATTGATCATATTATCATGCGTTCAAGTGCATTTTT

GGGTAT-3'
||||||
CCCATAGC-5'.
```

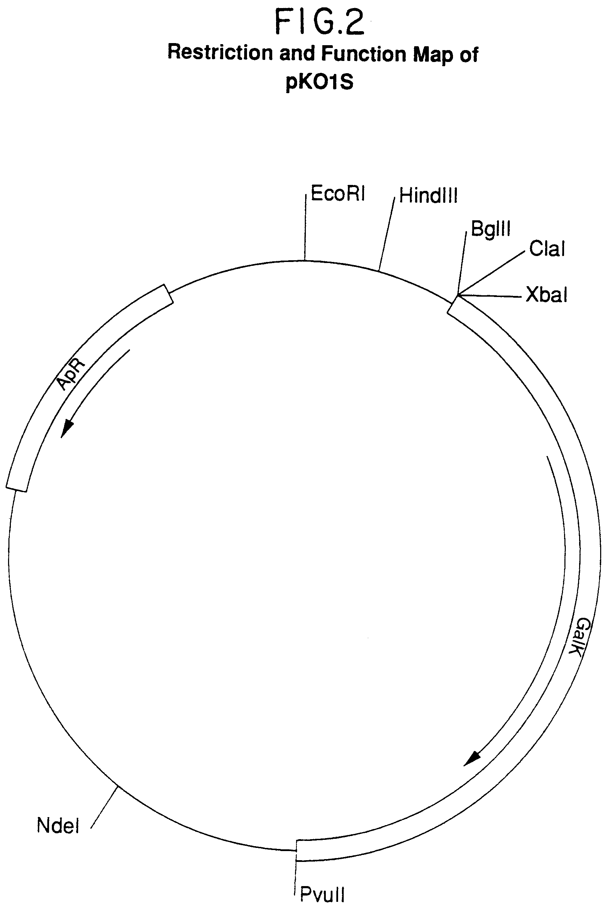

The transcriptional start sequence is denoted in the above DNA sequence by the *. The DNA sequences located 3' of this start site are the sequences found in the wild type tryptophan operon. The construction of plasmid pRB4-4 is described further in Example 1. A restriction and functional map of the plasmid is provided in Figure 2 of the accompanying drawings.

The 287 base pair EcoRI-ClaI restriction fragment of pRB4-4 was isolated and ligated into the 3.93 kb EcoRI-ClaI restriction fragment of the plasmid pK01S (construction of pK01S is described in Example 2). The resulting plasmid was pRB4-23. This plasmid has the Escherichia coli GalK gene operably linked to the novel transcriptional activating sequence of the present invention.

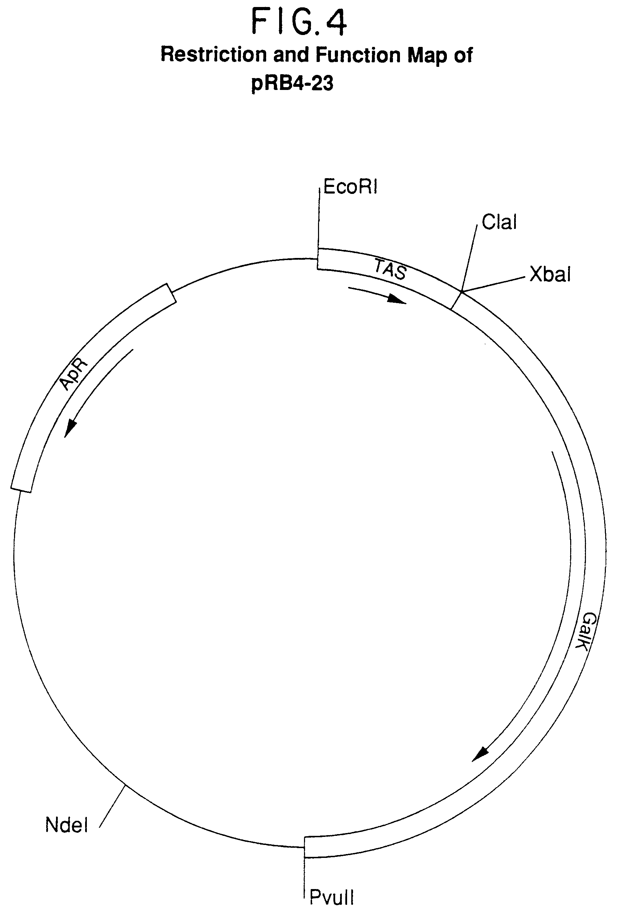

The construction of plasmid pRB4-23 is described further in Example 3. A restriction site and function map of the plasmid is provided in Figure 4 of the accompanying drawings.

The transcriptional activating sequence of the present invention provides an increase in the level of expression of an operably linked structural gene. For example, the transcriptional activating sequence present in pRB4-23 provides a 1.5 fold increase in the level of galactokinase expression as compared to the wild type tryptophan promoter.

The transcriptional activating sequence also remains regulatable. When transformants containing pRB4-23 are cultured in the presence of tryptophan, the repressor/tryptophan complex binds to the repressor binding site and expression of the galactokinase gene is repressed at a level comparable to that of the wild type Escherichia coli tryptophan promoter-operator region.

The invention includes expression vectors that contain a variety of elements used in conjunction with the regulatable transcriptional activating sequence of the present invention. For example, the vectors may be designed to express a variety of structural genes. Using cloning techniques that are well known in the art, other structural genes can be substituted for those that are specifically exemplified. Examples of such structural genes include: β-galactosidase, human pre-proinsulin, human proinsulin, human insulin A-chain, human insulin B-chain, non-human insulin, human growth hormone, insulin-like growth factors, human interferon, viral antigens, urokinase, tissue-type plasminogen activator, interleukins 1-6, colony stimulating factors, erythropoetin, human transferrin, and the like.

Also, the expression vectors of the present invention preferably contain a translational activating sequence. Translational activating sequences are described by Shine and Dalgarno, 1975, Nature 254:34 and Steitz, J.A. 1979, In Biological Regulation and Development: Gene Expression 1:349 (ed. R.F. Goldberg). Methods for constructing vectors containing translational activating sequences are described by Maniatis et al., 1989, Molecular Cloning: a Laboratory Manual (2nd edition) Cold Spring Harbor Laboratory Press, Cold Spring Harbor, N.Y. In pRB4-23 a translational activating sequence is present on the 3.96 kb EcoRI-ClaI restriction fragment from pKO1S in a position to provide translation of the GalK gene.

The transcriptional activating sequence and expression vectors of the present invention possess conveniently placed restriction enzyme sites. These restriction enzyme sites allow easy construction of derivative vectors that comprise the novel transcriptional activating sequence. The restriction enzyme sites can be modified to facilitate ligation if EcoRI and ClaI sites are not desired. The transcriptional activating sequence can be modified by adding, excluding or substituting nucleotides to provide a variety of unique or additional restriction enzyme sites.

There is much genetic and biochemical information about Escherichia coli that makes it a convenient and preferred bacterial host for purposes of the present invention. Moreover, since E. coli K12 is the host cell of choice for bioproduction of compounds of recombinant DNA technology, the applicability of the present invention to strains of that organism is distinctly advantageous. However, other organisms in which the transcriptional activating sequence of the present invention functions to provide well-regulated expression of a functional polypeptide are also within the scope of the invention.

Those skilled in the art will recognize that the present invention presents a method wherein a polypeptide product is expressed utilizing culture conditions that are well known in the art. The polypeptide product is then isolated from the resulting culture medium by routine methods.

The following examples further illustrate the invention disclosed herein. All enzymes referred to in the examples are available, unless otherwise indicated, from Bethesda Research Laboratories (BRL), P.O. Box 577, Gaithersburg, MD 20877 or New England Biolabs., Inc. (NEB), 32 Tozer Road, Beverly MA 01915, and are used in substantial accordance with the manufacturer's recommendations.

Example 1

Construction of Escherichia coli K12 RV308/pRB4-4

A. Preparation of the 4.34 kb EcoRI-ClaI Restriction Fragment from pBR322

Five µg of pBR322 DNA (available from BRL) was digested to completion with 2.5 µl (12.5 units) of ClaI

in a 100 μl reaction containing 50 mM NaCl, 6 mM Tris-HCl (pH 7.9) (Tris is Tris[hydroxymethyl]-aminomethane) and 6 mM MgCl$_2$. The mixture was incubated at 37°C for two hours. The digested DNA was extracted two times with a mixture of phenol and chloroform (50:50) and the aqueous layer was recovered. The DNA was recovered from the aqueous layer by addition of 2.5 volumes of absolute ethanol and 0.1 volume of 3 M sodium acetate. The DNA was collected by centrifugation and was resuspended in 10 μl of water.

The above digested DNA was digested further with EcoRI as follows. 10 μl of the ClaI-digested DNA was mixed with 2 μl (20 units) of EcoRI in a 100 μl reaction volume consisting of 50 mM Tris-HCl (pH 8.0), 10 mM MgCl$_2$ and 0.1 M NaCl. The mixture was incubated at 37°C for one hour. The DNA was precipitated with ethanol and sodium acetate as above, dried and resuspended in 20 μl of loading buffer (2.5% v/v glycerol, 0.005% w/v bromphenol blue, and 0.05% w/v xylene cyanole in TE buffer (10 mM Tris-HCl (pH 8.0) and 1 mM EDTA (EDTA is ethylenediamine tetraacetic acid)). The DNA was fractionated on a 1% low melting Sea Plaque agarose gel (FMC Corporation, Rockland, ME 02173) by gel electrophoresis as described by Maniatis et al. at pages 150-172 (Maniatis et al., 1982, Molecular Cloning: a Laboratory Manual, Cold Spring Harbor Laboratory, Cold Spring Harbor, N.Y.). The agarose gel was stained with a dilute solution of ethidium bromide and the 4.34 kb EcoRI-ClaI restriction fragment was visualized under a 300 nm UV light. The portion of the gel containing this restriction fragment was recovered as follows.

The gel piece was added to 5 mls of low salt buffer (0.2 M NaCl, 20 mM Tris-HCl (pH 7.5), and 1.0 mM EDTA) and melted at 65°C. The sample was added to an Elutip-d column (Schleicher and Schuell, Keane, N.H. 03431). The column was washed with 1-2 mls of high salt buffer (1.0 M NaCl, 20 mM Tris-HCl (pH 7.5) and 1.0 mM EDTA) using a 5 ml syringe and the equilibrated with 5 mls of low salt buffer. The DNA sample was loaded onto the column and then washed with an additional 5 mls of low salt buffer. The DNA was recovered by eluting with 1 ml of high salt buffer. Fractions of 0.4 ml were collected in a 1.5 ml tube and the DNA was precipitated with 1 ml of ethanol as described above. After drying, the DNA was resuspended in 30 μl of 10 mM Tris-HCl (pH 8.0).

## B. Preparation of the 229 Base Pair EcoRI-HincII Restriction Fragment

Twenty μg of pNM608 DNA was digested to completion with EcoRI and ClaI in substantial accordance with the method described in Example 1A. This digested DNA was purified and the small EcoRI-ClaI fragment was collected as described in Example 1A. This DNA was digested further with HincII as follows. Approximately 20 μg of the EcoRI-digested DNA was mixed with 5 μl (50 units) of HincII in a 100 μl reaction volume consisting of 10 mM Tris-HCl (pH 7.4), 7 mM MgCl$_2$, 100 mM NaCl, and 1 mM DTT. The mixture was incubated at 37°C for one hour. The resulting 229 base pair EcoRI-HincII fragment was isolated and purified by preparative gel electrophoresis in substantial accordance with the method of Example 1A.

## C. Preparation of the 58 Base Pair Linker DNA

The modified transcriptional activating sequence is shown in Figure 1B. This sequence is a 58 base pair fragment that comprises HincII and TaqI restriction enzyme recognition sequences at the 5′ and 3′ termini, respectively. This transcriptional activating sequence was prepared by the ligation of the five synthetic oligonucleotides shown below:

1. 5′-GACAACGAACTAGTTAACTAGTATAATAGTACG-3′
2. 5′-CAAGTTCACGTAAAAAAGGGTAT-3′
3. 5′-CTAGTTAACTAGTTCGTTGTC-3′
4. 5′-GAACTTGCGTACTATTATA-3′
5. 5′-CGATACCCTTTTTTACGT-3′

These oligonucleotides were synthesized on an automated DNA synthesizer (Applied Biosystems, 850 Lincoln Center Dr., Foster City CA. 94404) by the modified phosphotriester method using fully protected trideoxynucleotide building blocks in substantial accordance with the methods of Itakura et al., 1977, Science, 198:1058 and Crea et al., 1978, Pro. Natl. Acad. Sci. USA, 75:5765. The synthetic DNA segments were dissolved in 1 ml of TE buffer and stored at 0°C.

One nmole each of synthetic oligonucleotides 2-4 were phosphorylated by treatment with 1 μl (~ 10 units) of T4 polynucleotide kinase in 50 mM Tris-HCl (pH 7.6), 10 mM MgCl$_2$, 10 mM dithiothreitol (DTT) and 0.1 mM adenosine 5′-triphosphate (ATP) in a total volume of 100 μl for 30 minutes at 37°C. This incubation was followed by a 10 minute incubation at 65°C and subsequent freezing. The phosphorylated oligonucleotides were mixed with 1.2 nM of unphosphorylated oligonucleotides 1 and 5 in a reaction buffer containing 50 mM Tris-HCl (pH 7.6), 10 mM MgCl$_2$, 10 mM DTT, 50 mM ATP and 10 units of T4 DNA ligase. The reaction was incubated at 4°C overnight. After the incubation, the ligated 58 base pair double-stranded DNA fragment was purified by gel electrophoresis on a 15% polyacrylamide gel. The DNA fragment was cut out of the gel and was recovered by extraction with 2 M triethyl ammonium bicarbonate buffer (pH 7.9) followed by desalting on a DE-52 column as described by Brown et al. (Brown, E. et al., Methods in Enzymology 68:101). After isolation, the DNA fragment was phosphorylated with T4 polynucleotide kinase as described above. Following the kinase reaction, the DNA was passed through a Sephadex G-50 column (Pharmacia, P-L Biochemicals, Inc., 800 Centennial Avenue Piscataway, NJ 08854) and the isolated DNA was stored in 50 μl of 10 mM Tris-HCl (pH 8.0).

## D. Ligation and Transformation

One-half μg of the DNA from Example 1A was mixed with 1 μg of the DNA prepared in Example 1B and with 7.5 pmoles of synthetic DNA from Example 1C in a reaction buffer containing 50 mM Tris-HCl (pH 7.6), 10 mM MgCl$_2$, 10 mM DTT, 0.8 mM ATP and 5 units of T4 DNA ligase. The total reaction volume was 50 μl. After incubation at 40°C for 16 hours, 50 μl of 10 mM Tris-HCl (pH 7.6), and 3 μl of 1 M CaCl$_2$ were added. A portion of this reaction mixture was used to transform Escherichia coli K12 RV308 cells (NRRL B-15624) as described below.

Escherichia coli K12 RV308 cells were made competent for transformation as follows. A 50 ml culture of E. coli K12 RV308 was grown in L-broth (10 g tryptone, 10 g NaCl and 5 g yeast extract per liter of H$_2$O) to an O.D.$_{590}$ of 0.5 absorbance units. The culture was chilled on ice for ten minutes and then the cells were collected by centrifugation. The cell pellet was resuspended in 25 ml of cold 50 mM CaCl$_2$:10 mM Tris-HCl (pH 8.0) and incubated on ice for 15 minutes. The cells were collected by centrifugation, the cell pellet was resuspended in 2.5 ml of cold 50 mM CaCl$_2$:10mM Tris-HCl (pH 8.0) and the sample was held at 4°C for 16 hours.

Two hundred μl of this cell suspension was mixed with 50 μl of the ligated DNA prepared above and then incubated on ice for 60 minutes. The mixture was incubated at 32°C for 45 seconds and then placed on ice for 2 minutes. Five ml of TY medium (1% tryptone, 0.5% yeast extract and 1% sodium chloride, pH 7.4) was added to the mixture and incubation was continued at 32°C for 2 hours. One hundred μl of this culture was spread on TY agar plates (1% tryptone, 0.5% yeast extract, 1% sodium chloride and 1.5% agar at pH 7.4) that contained 50 μg/ml of ampicillin. These plates were incubated 16 hours at 32°C. The ampicillin resistant colonies were individually picked and used to inoculate 2 ml of TY medium. The cultures were incubated at 37°C with aeration for 16 hours.

Plasmid DNA was isolated from the culture of transformants as follows. All of the following manipulations were done at ambient temperature unless otherwise indicated. One and a half ml of each of the cultures was transferred to a microcentrifuge tube. The cells were collected by a 10 second centrifugation. The supernatant was removed with a fine-tip aspirator and the cell pellet was suspended in 100 μl of freshly prepared lysozyme solution containing 2 μg/ml lysozyme, 50 mM glucose, 10 mM EDTA and 25 mM Tris-HCl (pH 8.0). After incubation at 0°C for 10 minutes, 200 μl of an alkaline sodium dodecyl sulfate (SDS) solution (0.2 N NaOH, 1% SDS) was added. The tube was gently vortexed and then maintained at 0°C for 15 minutes. Next, 150 μl of 3 M sodium acetate (prepared by dissolving 3 moles of sodium acetate in a minimum of water, adjusting the pH to 4.8 with glacial acetic acid and then adjusting the volume to 1 liter) were added and the contents of the tube mixed gently by inversion. The sample was kept at 0°C for 10 minutes and then centrifuged for 5 minutes to yield an almost clear supernatant. One-third of the supernatant was transferred to a second centrifuge tube to which 1 ml of cold absolute ethanol was added. After the tube was held at -20°C for 30 minutes, the resultant precipitate was collected by centrifugation and the supernatant was removed by aspiration. The pellet was dissolved in 100 μl of 0.1 M sodium acetate/0.05 M Tris-HCl (pH 8.0) and then re-precipitated by the addition of 2 volumes of cold absolute ethanol. The resultant precipitate was collected by centrifugation, rinsed with 70% ethanol, dried, dissolved in water and stored at 0°C.

## Example 2

## Construction of the Plasmid pKO1S

### A. SmaI Digestion of pKO1

Five μg of the plasmid pK01 (available from P-L Biochemicals, Inc. 1037 West McKinley Avenue, Milwaukee, WI 53205, cat. # 27-4932) was digested to completion in a 100 μl reaction volume containing 1 μl (10 units) of SmaI, 6 mM Tris-HCl (pH 8.0), 6 mM MgCl$_2$, 20 mM KCl and 6 mM β-mercaptoethanol at 25°C for 1 hour. After the incubation, the DNA was extracted and precipitated as described in Example 1A.

7

B. Construction of BglII-ClaI-XbaI Linker

A synthetic oligonucleotide linker was prepared in substantial accordance with the method described in Example 1C. The following oligonucleotides were synthesized:

1) 5′-TCTAGAATCGATAGATCT-3′ and
2) 5′-AGATCTATCGATTCTAGA-3′.

Fifty pmoles of each oligonucleotide were mixed together and incubated at 95°C for 15 minutes in a 50 µl volume. The sample was allowed to slowly cool to room temperature allowing the oligonucleotides to anneal and form a double stranded molecule. This linker contains BglII, ClaI, and XbaI restriction enzyme cleavage sites.

C. Ligation and Transformation

Five pmoles of the annealed linker from Example 2B and 1 µg of the SmaI digested pKO1 plasmid DNA prepared in Example 2A were ligated with T4 DNA ligase in accordance with the procedure of Example 1D. A portion of this ligation mix was used to transform Escherichia coli K12 RV308 cells in accordance with the method described in Example 1D. The transformants were selected on TY agar plates containing 50 µg/ml of the antibiotic ampicillin and grown for 16 hours at 37°C in TY broth containing 50 µg/ml of ampicillin. Plasmid DNA was isolated from the ampicillin resistant clones according to the method described in Example 1D. The plasmid DNA was characterized by restriction analysis. pKO1S contained the linker DNA cloned into the SmaI site of pKO1.

Example 3

Construction of Escherichia coli K12 RV308/pRB4-23

A. Isolation of the 3932 Base Pair EcoRI-ClaI Restriction Fragment from Plasmid pK01S

Ten µg of plasmid pK01S was digested with ClaI and EcoRI in substantial accordance with the procedures described in Example 1A. Following digestion, the DNA was fractionated by gel electrophoresis and the 3932 base pair EcoRI-ClaI restriction fragment was isolated from the gel in accordance with the methods described in Example 1A.

B. Isolation of the 287 Base Pair EcoRI-ClaI Fragment from Plasmid PRB4-4

Ten µg of plasmid pRB4-4 was digested to completion with the restriction enzymes EcoRI and ClaI in substantial accordance with the methods described in the Example 3A. The sample was fractionated on a 1% low melting agarose gel and the 287 base pair fragment was isolated and purified in accordance with the methods of Example 1A.

C. Ligation and Transformation

One-half µg of the DNA from Example 3A was mixed with 100 ng of the DNA from Example 3B in a reaction buffer containing 50 mM Tris-HCl (pH 7.6), 10 mM MgCl , 10 mM DTT, 0.8 mM ATP and 3 units of T4 DNA ligase in a reaction volume of 50 µl. The mixture was incubated at 4°C for 16 hours and then diluted with 50 µl of 10 mM Tris-HCl (pH 7.6) and 3 µl of 1 M CaCl$_2$. Fifty µl of this reaction mixture was used to transform Escherichia coli K12 RV308 cells in accordance with the method described in Example 1D. Transformants were selected on TY agar plates containing 50 µg/ml ampicillin. Plasmid DNA was isolated from the ampicillin resistant transformants by the alkaline lysis method described in Example 1D. The plasmid DNA was examined by restriction analysis. The plasmid pRB4-23 contained a 287 base pair EcoRI-ClaI fragment. In this manner, the E. coli K12 RV308/pRB4-23 transformants were isolated.

Example 4

Analysis of Gene Expression

The level of gene expression from the transcriptional activating sequence was analyzed by measuring galactokinase gene expression from pRB4-23. Galactokinase expression was compared to the plasmid, pRB4-

8

23A, that contains the wild type tryptophan operon promoter-operator transcriptional activating sequence operably linked to the galactokinase gene.

Escherichia coli K12 RV308/pRB4-23 and E. coli K12 RV308/pRB-23A were individually cultured 16 hours at 37°C in 5 mls of M9 medium (200 mls of 5x M9 salts (consisting of 64 g/l $Na_2HPO_4$-$7H_2O$, 15 g/l $KH_2PO_4$, 2.5 g/l NaCl and 5.0 g/l $NH_4Cl$) and 25 mls/l of 20% glucose) containing 12.5 μg/ml thymidine, 0.5% casimino acids, 100 μg/ml tryptophan and 100 μg/ml ampicillin. Following incubation, the cultures were diluted 50X in M9 medium lacking tryptophan and grown for four hours at 37°C until an absorbance ($A_{550}$) of 0.7-1.0 was reached. One ml samples of the cultures were harvested by centrifugation. The cell pellets were resuspended in 50-100 μl of loading buffer (50 mM Tris-HCl (pH 6.8), 100 mM DTT, 2% SDS, 0.1% bromphenol blue and 10% glycerol). Samples were boiled for 5 minutes and 10-20 μl of the samples were analyzed by electrophoresis on a 12.5% polyacrylamide gel. The gels were stained with Coomassie blue and analyzed by densitometry using a Schimadzu (Kyoto, Japan) Dual Wavelength TLC Scanner (model CS-930) on line with a Schimadzu Data Recorder (model DR-2).

Alternatively, galactokinase assays were performed on the samples to determine the level of galactokinase expression. These assays were performed essentially as described by McKenney et al., 1981, in Gene Amplification and Analysis, Chirikjan and Papas, volume 2, pages 384-408, with slight modifications. Essentially, the cells containing plasmids pRB4-23 and pRB4-23A were grown to log phase ($OD_{650}$ = 0.6) in M9 media (200 mls of 5x M9 salts (consisting of 64 g/l $Na_2HPO_4$·$7H_2O$, 15 g/l $KH_2PO_4$, 2.5 g/l NaCl and 5.0 g/l $NH_4Cl$) and 20 mls/l of 20% fructose) containing 5 μg/ml ampicillin. A 1.0 ml sample was removed to a screw cap tube and 40 μl of lysis buffer (100 mM EDTA, 100 mM DTT and 50 mM Tris-HCl, pH 8.0) and a few drops of toluene were added. Samples were vigorously vortexed, then placed in a 37°C shaker until toluene was completely evaporated off. A 5 μl sample was added to 80 μl of a reaction mixture consisting of 20 μl of mix #1 (5mM DTT, 16 mM NaF), 50 μl of mix #2 (8 mM $MgCl_2$, 200 mM Tris-HCl pH 7.9, 3.2 mM ATP) and 10 μl of $^{14}$C-galactose, available from Amersham, Inc., Arlington Heights, IL 60005 (specific activity was adjusted to 4.5 x $10^6$ dpm per μmole). The reaction mixture was incubated at 37°C for five minutes and an aliquot of 25 μl was spotted on Whatman DE-81 paper strip (1 x 30 cm). The strip was run by descending chromatography using water as the solvent. At the end, the strip was dried and counts (cpm) at the origin of loading were measured by the Beckman (Palo Alto, CA 94304) Liquid Scintillation System, model LS-7000.

In the presence of tryptophan, galactokinase gene expression by PRB4-23 was regulated at a level comparable to that of the wild type tryptophan operon transcriptional activating sequence of pRB4-23A. When grown in the absence of tryptophan, gene expression by pRB4-23 was at a level 1.5 times greater than that of pRB4-23A.

## Claims

1. A tryptophan operon transcriptional activating sequence comprising a -10 consensus sequence and a wild type repressor binding site.

2. A recombinant DNA expression vector which comprises the transcriptional activating sequence of Claim 1.

3. A recombinant DNA expression vector which sequentially comprises
   a) the transcriptional sequence of Claim 1,
   b) a translational activating sequence and
   c) a DNA sequence that codes for a functional polypeptide;
   such that sequences a and b are positioned for the expression of sequence c.

4. A recombinant DNA expression vector of Claim 3 that is a plasmid.

5. A host cell transformed with the recombinant DNA expression vector of Claim 2, 3 or 4.

6. The tryptophan operon transcriptional activating sequence of Claim 1 of the structure:

```
5' - GTTGACAACGAACTAGTTAACTAGTATAATAGTACGCA - 3'
     ||||||||||||||||||||||||||||||||||||||
3' - CAACTGTTGCTTGATCAATTGATCATATTATCATGCGT - 5'
```

wherein A is deoxyadenyl, G is deoxyguanyl, C is deoxycytidyl and T is thymidyl.

7. A recombinant DNA expression vector which comprises the transcriptional activating sequence of Claim 6.

8. A recombinant DNA expression vector which sequentially comprises:
   a) the transcriptional activating sequence of Claim 6,
   b) a translational activating sequence and
   c) a DNA sequence that codes for a functional polypeptide;
   such that sequences a and b are positioned for the expression of sequence c.

9. A host cell transformed with a recombinant DNA expression vector of Claim 7 or 8.

10. A process for preparing a functional polypeptide, which comprises culturing the host cell of Claim 5 or 9 under culture conditions that enable the expression of said polypeptide.

**Claims for the following Contracting State: ES**

1. A process for preparing a functional polypeptide, said process comprising:
   a) preparing a recombinant DNA expression vector which sequentially comprises:
      i) a tryptophan operon transcriptional activating sequence comprising a -10 consensus sequence and a wild type repressor binding site,
      ii) a translational activating sequence, and
      iii) a DNA sequence that codes for a functional polypeptide; such that sequences i and ii are positioned for the expression of sequence iii;
   b) transforming said recombinant DNA expression vector into a host cell; and
   c) culturing the transformed host cell under conditions that allow expression of said functional polypeptide.

2. A process according to Claim 1 in which the recombinant DNA expression vector comprises a tryptophan operon transcriptional activating sequence of the structure:

```
5' - GTTGACAACGAACTAGTTAACTAGTATAATAGTACGCA - 3'
     | | | | | | | | | | | | | | | | | | | | | | | | | | | | | | | | | | | | | |
3' - CAACTGTTGCTTGATCAATTGATCATATTATCATGCGT - 5'
```

wherein A is deoxyadenyl, G is deoxyguanyl, C is deoxycytidyl and T is thymidyl.

3. A process according to Claim 1 or 2 in which the recombinant DNA expression vector is a plasmid.

4. A process according to Claim 1, 2 or 3 in which the host cell is Escherichia coli.

# FIG.I

A. Wild type tryptophan transcriptional activating sequence

REPRESSOR BINDING SITE

5'-GTTGACAATTAATCATCGAACTAGTTAACTAGTACGCA-3'

     **▬▬▬▬▬**                  **▬▬▬▬**      * mRNA start

    -35 region                    -10 region

B. Modified tryptophan transcriptional activating sequence

REPRESSOR BINDING SITE

5'-GTTGACAACGAACTAGTTAACTAGTATAATAGTACGCA-3'

     **▬▬▬▬▬**                   **▬▬▬▬**      * mRNA start

    -35 region                    -10 region

# FIG.2
## Restriction and Function Map of
## pKO1S

# F I G.3
## Restriction and Function Map of
## pRB-4

# FIG.4

## Restriction and Function Map of
## pRB4-23